# EUROPÄISCHE PATENTSCHRIFT

(11) **EP 0 585 678 B1**
(45) Veröffentlichungstag und Bekanntmachung des Hinweises auf die Patenterteilung: **17.06.1998**
(21) Anmeldenummer: 93112912.6
(22) Anmeldetag: 12.08.1993
(51) Int. Cl.: C07D 307/94, C07C 31/20, C08G 65/02

(54) **Verfahren zur Herstellung von Reduktionsprodukten von Derivaten der 4-Hydroxybuttersäure**
Process of preparing reduction products of 4-hydroxybutyric acid derivatives
Procédé de préparation de produits de réduction de dérivés de l'acide 4-hydroxybutyrique

(30) Priorität: 29.08.1992 DE 4228884
(43) Veröffentlichungstag der Anmeldung: 09.03.1994
(73) Patentinhaber: BASF Aktiengesellschaft, 67063 Ludwigshafen (DE)
(72) Erfinder: Weyer, Hans-Juergen, Dr., D-6800 Mannheim 1 (DE); Fischer, Rolf, Dr., D-6900 Heidelberg (DE); Schnurr, Werner, Dr., D-6719 Herxheim (DE); Goetz, Norbert, Dr., D-6520 Worms 1 (DE); Kuekenhoehner, Thomas, Dr., D-6737 Boehl-Iggelheim (DE)

(56) Entgegenhaltungen:
- EP-A- 0 284 969
- EP-A- 0 343 985
- EP-A- 0 431 923
- DE-A- 2 414 253
- US-A- 3 956 318
- US-A- 4 301 077
- DATABASE WPI Week 7414, Derwent Publications Ltd., London, GB; AN 74-25928 & JP-B-49 009 464 (MITSUBISHI CHEM. INDS. LTD) 5. März 1974
- HOUBEN-WEYL 'Band 4/1c, Reduktion' 1980 , THIEME VERLAG , STUTTGART DE * Seite 404, 2. alpha) - Seite 410 *
- ANGEWANDTE CHEMIE. INTERNATIONAL EDITION. Bd. 23, Nr. 12 , 1984 , WEINHEIM DE Seiten 980 - 981 M. HUHTASAARI ET AL. 'Cyclization of 3-Allyloxycarboxylic Acids to Tetrahydrofurans by Kolbe Electrolysis'
- DATABASE WPI Week 7541, Derwent Publications Ltd., London, GB; AN 75-68078 & JP-A-50 084 505 (SAGAMI CHEM. RES. CENTRE) 8. Juli 1975
- BULLETIN OF THE CHEMICAL SOCIETY OF JAPAN Bd. 44, Nr. 9 , September 1971 , TOKYO JP Seiten 2473 - 2479 T. SAEGUSA ET AL. 'The Acid-catalyzed Reaction of Isocyanide with Oxetane'

## Beschreibung

Die vorliegende Erfindung betrifft ein Verfahren zur Herstellung von Reduktionsprodukten (I) von Derivaten der 4-Hydroxybuttersäure der allgemeinen Formel IIa 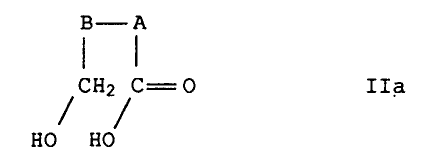 in der einer der Reste A und B eine Methylengruppe und der andere eine Cyclopropylidengruppe bedeutet, oder von C₁-C₄-Alkylestern (IIb) oder von Lactonen (IIc) dieser Säuren IIa. Weiterhin betrifft die Erfindung neue Reaktionsprodukte von II sowie Polymere, die aus diesen neuen Verbindungen hergestellt werden können.

Im einzelnen handelt es sich bei den Reaktionsprodukten I um folgende Verbindungen: 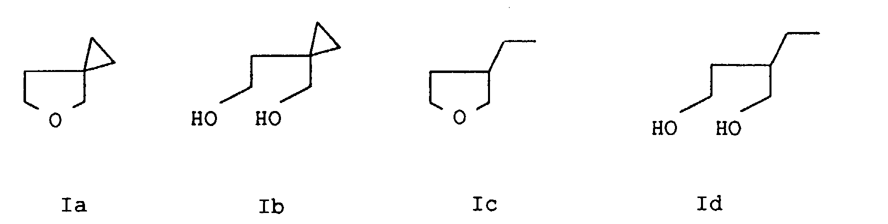 2-substituierte Butandiole haben bekanntlich als Bausteine für Polyester Bedeutung, während 3-substituierte Tetrahydrofurane als Comonomere für die Copolymerisation mit Tetrahydrofuran dienen. So dient 3-Methyltetrahydrofuran zur Herstellung von Polymeren, aus denen elastische Fasern gefertigt werden (EP-A-343 985). Verbindungen mit Substituenten, die von Methyl verschieden sind, sind bisher schlecht zugänglich oder im Falle der Verbindungen Ia und Ib unbekannt.

Einen Zugang zu 3-Hydroxymethyl-pentanol Id bietet die Hydrierung von 4-Ethyl-3,6-dihydro-1,2-dioxin nach der Lehre der JP-A 50/84 505.

3-Ethyltetrahydrofuran Ic kann durch katalytische Hydrierung von 3-Ethyl-3,4-epoxybutanol nach der Lehre der US-A 3 956 318 hergestellt werden. Die JP-A 49/9463, die JP-A 49/9464 und die JP-A 50/1038 lehren die Herstellung von 3-Alkyltetrahydrofuran aus Alkylmaleinsäuren. Allen Ausgangsverbindungen ist gemeinsam, daß sie nur aufwendig hergestellt werden können.

M. Huhtasaari et al., Angew. Chem. Int. Ed. Engl. **23** (12) (1984) 980-981, beschreiben die Darstellung von 2-Ethyltetrahydrofuran durch Kolbe-Elektrolyse von 3-Allyloxicarbonsäuren. Dieses Verfahren ist relativ umständlich, wirtschaftlich nur wenig attraktiv und die Ausgangsstoffe sind in technischen Mengen nur schwierig erhältlich.

Aus EP-A-431 923 ist bekannt, daß man γ-Butyrolacton in Gegenwart von Cu-Katalysatoren bei 150°C bis 300°C zu Butandiol und THF hydrieren kann. Diese Schrift gibt keinen Hinweis auf die Herstellung 3-substituierter Tetrahydrofurane oder 2-substituierter Butandiole.

In Houben-Weyl: "Methoden der organischen Chemie", Band 4/1c, Stuttgart 1980, Seiten 404-410, ist das Verhalten zahlreicher eine Cyclopropyleinheit enthaltender Verbindungen unter hydrierenden Bedingungen beschrieben. Aus dieser Literaturstelle ist nicht abzuleiten, in welcher Weise Derivate der 4-Hydroxibuttersäure mit der allgemeinen Formel IIa unter hydrierenden Bedingungen in Gegenwart von Cobalt-, Nickel- und Kupferkatalysatoren reagieren würden.

Es bestand die Aufgabe, ein Verfahren zur Verfügung zu stellen, das zu neuen substituierten Tetrahydrofuranen oder den entsprechenden Diolen führt. Weiterhin soll es in der Lage sein, an sich bekanntes 3-ethylsubstituiertes Tetrahydrofuran besser zugänglich zu machen.

Diese Aufgabe wurde gelöst, indem ein Verfahren zur Herstellung der Verbindungen I aus den Verbindungen II gefunden wurde, das dadurch gekennzeichnet ist, daß man die Verbindungen IIa bzw. IIb bzw. IIc mit Wasserstoff in Gegenwart eines heterogenen Hydrierkatalysators hydriert.

Weiterhin werden die neuen Verbindungen Ia und Ib sowie Polymere, die aus Ia aufgebaut sind, bereitgestellt.

Die Ausgangsverbindungen IIa und IIb sind nach an sich bekannten Methoden aus den Verbindungen IIc erhältlich. 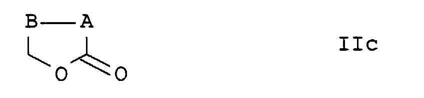 Bevorzugt werden Verbindungen, in denen A die Cyclopropylidengruppe und B die Methylengruppe bedeuten.

So ist 5-Oxa-[2,4]-spiro-heptanon-6 aus Bull. Chem. Soc. Jap. 44 (1971) 2473 bekannt. Bevorzugt wird aber 5-Oxa-[2,4]-spiro-heptanon-4 (IIc') 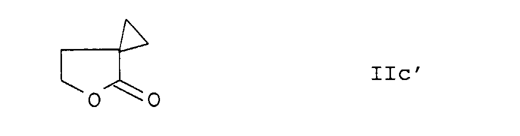 IIc' fällt bei der Herstellung von Tetrahydropyran-4-carbonsäuremethylester in relativ großer Menge an, wie es in der EP-A 284 969 beschrieben wird. Bei der dort in Beispiel 1 als 3-Vinylbutyrolacton benannten Verbindung handelt es sich tatsächlich um IIc'.

Die Ausgangsverbindungen II werden an Hydrierkatalysatoren umgesetzt. Dieses sind allgemein übliche Katalysatoren, wie sie z.B. in Houben-Weyl, Methoden der organischen Chemie, Bd. 4/1c, S. 15 bis 28, Thieme Verlag, 1980 beschrieben sind. Bevorzugte Hydrierkatalysatoren sind solche, die Kupfer und/oder Metalle der Gruppen 8 bis 10 gemäß der IUPAC-Einteilung des Periodensystems enthalten, wovon wiederum Cobalt, Nickel, Ruthenium, Rhodium, Palladium und Platin bevorzugt werden. Die Katalysatoren können ohne Träger oder auf einem Träger verwendet werden, wobei die Art des Trägers in der Regel nicht kritisch ist. Es kommen daher übliche Trägermaterialien wie Siliciumdioxid, Aluminiumoxid, Titanoxid, Aktivkohle, Silikate und Zeolithe in Betracht.

Zur Herstellung der Trägerkatalysatoren können Bindemittel oder Formhilfsmittel mitverwendet werden. Solche Katalysatoren können in Form von Splitt, Strängen oder Kugeln eingesetzt werden.

Es haben sich Katalysatorbelastungen von 0,01 bis 1, insbesonderé von 0,05 bis 0,3 kg II pro Liter Katalysator und Stunde bewährt.

Die Hydrierung kann in Gegenwart eines Lösungsmittels erfolgen. Geeignet sind polare Lösungsmittel wie Ether, Alkohole oder Wasser oder Mischungen hiervon. Bevorzugt sind Ether und Alkohole mit bis zu sechs Kohlenstoffatomen wie Ethanol, n-Propanol, iso-Propanol und n-Butanol.

Die Temperatur kann bei der Hydrierung 50 bis 400°C, insbesondere 150 bis 300°C betragen. Der Druck ist in weiten Grenzen wählbar, die von 1 bis 400 bar reichen, insbesondere von 50 bis 300 bar. Das Verfahren kann kontinuierlich oder diskontinuierlich betrieben werden. Als Reaktoren eignen sich z.B. Rohrreaktoren oder Rohrbündelreaktoren.

Das erfindungsgemäße Verfahren läßt sich vorteilhaft zur Herstellung überwiegender Mengen Ib oder Ic nutzen.

So läßt sich Ic im Reaktionsaustrag stark anreichern, wenn IIc' bei Temperaturen von 200 bis 400°C und in Gegenwart saurer Verbindungen hydriert wird. Diese sauren Verbindungen umfassen Protonensäuren wie Schwefelsäure, Chlorwasserstoffsäure und Phosphorsäure, Lewis-Säuren wie Bortrifluorid und Zinkchlorid sowie weiterhin Zeolithe, Kationentauscher, Silikate, Aluminiumoxide, Aluminiumphosphate und andere saure Metalloxide. Diese Verbindungen können dem Reaktionsgemisch zugesetzt werden. Vorteilhaft ist aber die Anwendung der sauren Verbindungen mit dem Katalysator in der Form, daß die katalytisch aktiven Komponenten auf einen sauer wirkenden Träger aufgebracht werden.

Verbindung Ib läßt sich im Reaktionsaustrag stark anreichern, wenn IIc' bei Temperaturen von 50 bis 200°C an neutralen Katalysatoren hydriert wird.

Die Isolierung der Reaktionsprodukte I geschieht nach bekannten Methoden, vorzugsweise destillativ.

Verbindung Ia läßt sich durch kationische Polymerisation in Polymere überführen. Als copolymerisierbare Monomere sind vor allem Tetrahydrofuran und 3-Methyltetrahydrofuran zu nennen.

Das erfindungsgemäße Verfahren erlaubt die Herstellung eines neuen 3-substituierten Tetrahydrofurans und des entsprechenden Diols sowie die Synthese von 3-ethylsubstituiertem Tetrahydrofuran in guter Ausbeute.

### Beispiele

### Katalysatorzusammensetzung

Alle Prozentangaben sind Gewichtsprozente.
- Katalysator A: 67 % CoO, 19,8 % CuO, 7 % Mn₂O₃, 3 % MoO₃, 0,2 % Na₂O, 3 % H₃PO₄ (nach EP-B 100 406; S. 2, Zeile 63-S. 3, Zeile 11)
- Katalysator B: 50 % NiO, 17 % CuO, 31 % γ-Al₂O₃, 2 % MoO₃ (nach EP-A 18 569, S. 2, Zeile 27-S. 3, Zeile 18)
- Katalysator C: 36,5 % CuO, 1,0 % BaO, 0,6 % Cr₂O₃, 0,4 % ZnO, 14,4 % MgO, 28,5 % SiO₂, 18,6 % H₂O (nach DE-A 869 052, S. 1-S. 2, Zeile 15)
- Katalysator D: 40 % CuO, 40 % ZnO, 20 % γ-Al₂O₃, 0,1 % Na₂O (nach DE-A 1 542 632, S. 2-S. 4, Zeile 2)
- Katalysator E: 56 % CuO, 44 % γ-Al₂O₃ (nach EP-A 44 444, S. 8, Zeile 33-S. 11, Zeile 1)

### Beispiele 1 bis 9

10 g IIc' wurden in 100 ml n-Butanol in Gegenwart von 20 ml Katalysator (2,5 bis 4 mm Splitt) bei 200 bar Wasserstoffdruck und verschiedenen Temperaturen T diskontinuierlich hydriert. Nach Ende der Reaktion wurden die Reaktionsausträge gaschromatographisch analysiert.

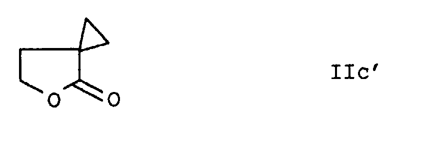

| Bsp. | Katalysator | T[°C] | Ausbeute* | | | |
|---|---|---|---|---|---|---|
| | | | Ia | Ib | Ic | Id |
| 1 | A | 150 | 0 | 84 | 2 | 14 |
| 2 | B | 175 | 5 | 23 | 71 | 1 |
| 3 | C | 150 | 0 | 93 | 0 | 7 |
| 4 | D | 175 | 1 | 87 | 1 | 11 |
| 5 | D | 200 | 2 | 55 | 11 | 32 |
| 6 | D | 225 | 1 | 0 | 94 | 6 |
| 7 | E | 150 | 1 | 88 | 2 | 9 |
| 8 | E | 175 | 22 | 46 | 23 | 3 |
| 9 | E | 200 | 27 | 0 | 73 | 0 |

| | | | | | | |
|---|---|---|---|---|---|---|
| * Neben den Verbindungen Ia bis Id enthielt der Reaktoraustrag geringe Mengen nicht identifizierter Verbindungen. | | | | | | |

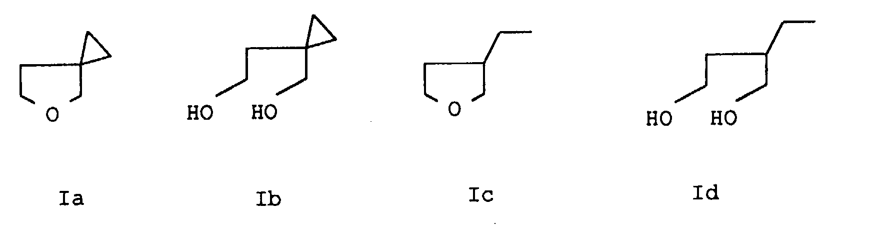

### Beispiel 10

An 18 g Katalysator C in Form von 2,5 bis 4 mm großem Splitt in einem Festbett wurden stündlich 3,6 g IIc' in 14 ml Methanol in Rieselfahrweise bei 200°C und 200 bar Wasserstoffdruck bei einer Katalysatorbelastung von 0,12 kg IIc'/Liter Katalysator und Stunde umgesetzt. Die Ausbeute an Ib betrug 45 %, die an Ic 35 %. Der Umsatz von IIc' war quantitativ.
5-Oxa-[2,4]-spiroheptan
Sdp. 112°C/1013 mbar
¹H-NMR (CDCl₃) : 3,97 (2H, t, J = 7 Hz) ; 3,64 (2H, s); 1,88 (2H, t, J = 7 Hz); 0,68 - 0,65 (2H, m), 0,62 - 0,59 ppm (2H, m).
¹³C-NMR (CDCl₃): 75,0 (CH₂); 68,7 (CH₂); 35,4 (CH₂); 22,4 (C);
10,8 ppm (2 x CH₂).
2-(1-Hydroxymethyl-cyclopropyl)ethanol Ib:
Sdp. 113°C/1 mbar
¹H-NMR (CDCl₃): 4,77 (2H, s, OH); 3,68 (2H, t, J = 5Hz); 3,38 (2H, s); 1,60 (2H, t, J = 5Hz); 0,48 (2H, dd, J₁ = 6 Hz, J₂ = 4Hz); 0,38 ppm (2H, dd, J₁ = 6Hz, J₂ = 4 Hz).
¹³C-NMR (CDCl₃): 69,9 (CH₂); 61,1 (CH₂); 39,8 (CH₂); 21,7 (C); 10,9 ppm (2 x CH₂).

## Patentansprüche

1. Verfahren zur Herstellung von Reduktionsprodukten (I) von Derivaten der 4-Hydroxybuttersäure der allgemeinen Formel IIa 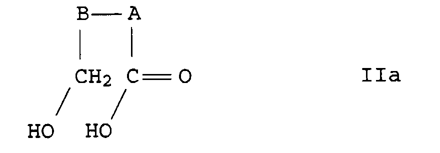 in der einer der Reste A und B eine Methylengruppe und der andere eine Cyclopropylidengruppe bedeutet, oder von C₁-C₄-Alkylestern (IIb) oder von Lactonen (IIc) dieser Säuren IIa, dadurch gekennzeichnet, daß man die Verbindungen IIa bzw. IIb bzw. IIc mit Wasserstoff in Gegenwart eines heterogenen Hydrierkatalysators katalytisch hydriert.

2. Verfahren nach Anspruch 1, dadurch gekennzeichnet, daß man von einer Verbindung IIc ausgeht, in der A die Cyclopropylidengruppe und B die Methylengruppe bedeuten.

3. Verfahren nach Anspruch 1 oder 2, dadurch gekennzeichnet, daß man Katalysatoren verwendet, die Kupfer und/oder Metalle der Gruppen 8 bis 10 des Periodensystems enthalten.

4. 5-Oxa-[2,4]-spiroheptan

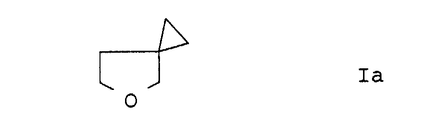

5. 2-(1-Hydroxymethyl-cyclopropyl)ethanol

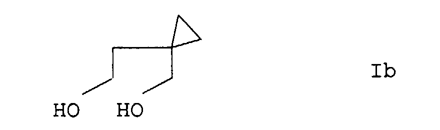

6. Verwendung der Verbindungen gemäß Anspruch 4 und/oder Anspruch 5 zur Herstellung von Polyethern und/oder Polyestern.

## Claims

1. A process for the preparation of products (I) of the reduction of 4-hydroxybutyric acid derivatives of the formula IIa 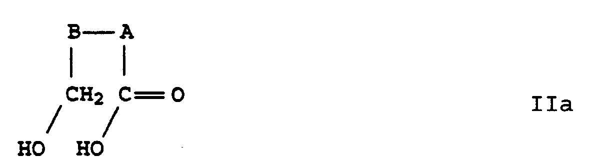 where one of A and B is methylene and the other is cyclopropylidene, or of C₁-C₄-alkyl esters (IIb) or lactones (IIc) of these acids IIa, which comprises hydrogenating the compounds IIa or IIb or IIc catalytically using hydrogen in the presence of a heterogeneous hydrogenation catalyst.

2. A process as claimed in claim 1, wherein a compound IIc is used where A is cyclopropylidene and B is methylene.

3. A process as claimed in claim 1 or 2, wherein a catalyst is used which contains copper and/or metals from groups 8 to 10 of the Periodic Table.

4. 5-Oxa[2,4]spiroheptane

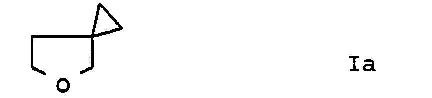

5. 2-(1-Hydroxymethylcyclopropyl)ethanol

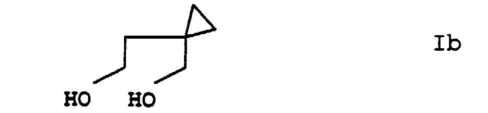

6. The use of a compound as claimed in claim 4 and/or claim 5 for the preparation of polyethers and/or polyesters.

## Revendications

1. Procédé de préparation de produits (I) de la réduction de dérivés de l'acide 4-hydroxybutyrique de formule générale IIa 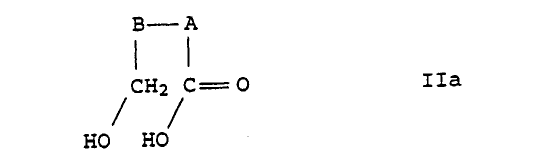 dans laquelle l'un des restes A et B représente un groupement méthylène et l'autre un groupement cyclopropylidène, ou d'esters d'alkyle en C₁-C₄ (IIb) ou de lactones (IIc) de ces acides IIa, caractérisé en ce que l'on hydrogène de façon catalytique les composés IIa, respectivement IIb, respectivement IIc, avec de l'hydrogène en présence d'un catalyseur d'hydrogénation.

2. Procédé selon la revendication 1, caractérisé en ce que l'on part d'un composé IIc dans lequel A représente le groupement cyclopropylidène et B le groupement méthylène.

3. Procédé selon la revendication 1 ou 2, caractérisé en ce que l'on utilise des catalyseurs contenant du cuivre et/ou des métaux des groupes 8 à 10 de la classification périodique des éléments.

4. 5-oxaspiro[2.4]heptane

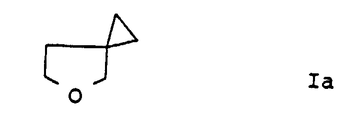

5. 2-(1-hydroxyméthyl-cyclopropyl)éthanol

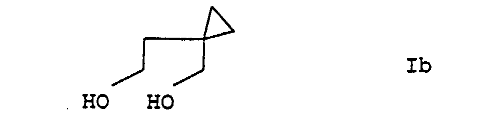

6. Utilisation des composés selon la revendication 4 et/ou la revendication 5 pour la préparation de polyéthers et/ou de polyesters.
